# EUROPEAN PATENT APPLICATION

(11) **EP 0 967 289 A1**
(43) Date of publication of application: **29.12.1999**
(21) Application number: 98401108.0
(22) Date of filing: 06.05.1998
(51) Int. Cl.: C12N 15/87, A61K 48/00

(54) **Improvements in gene therapy using compositions comprising a polynucleotide and a nuclease inhibitor**

(71) Applicant: TRANSGENE S.A., 67082 Strasbourg Cédex (FR); ASSOCIATION FRANCAISE CONTRE LES MYOPATHIES, F-75013 Paris (FR)
(72) Inventor: Braun, Serge, 67120 Dorlisheim (FR)
(74) Representative: VOSSIUS & PARTNER

(57) **Abstract**

Described is the use of a nuclease inhibitor for the preparation of a therapeutic composition for the introduction of a polynucleotide into a cell as well as a composition comprising a nuclease inhibitor and at least one polynucleotide.

## Description

The present invention relates to the use of a nuclease inhibitor for the preparation of a therapeutic composition for improving transfection of a polynucleotide into a cell, and to related compositions comprising a polynucleotide and such a nuclease inhibitor. Such a composition is useful in gene therapy, vaccination, and any therapeutic situation in which a gene-based product is administered to cells *in vivo*.

Gene therapy has generally been conceived as principally applicable to heritable deficiency diseases (cystic fibrosis, dystrophies, haemophilias, etc.) where permanent cure may be effected by introducing a functional gene. However, a much larger group of diseases, notably acquired diseases (cancer, AIDS, multiple sclerosis, etc.) might be treatable by transiently engineering host cells to produce beneficial proteins.
Applications are, for example, the treatment of muscular dystrophies or of cystic fibrosis. The genes of Duchenne/Becker muscular dystrophy and cystic fibrosis have been identified and encode polypeptides termed dystrophin and cystic fibrosis transmembrane conductance regulator (CFTR), respectively. Direct expression of these genes within, respectively, the muscle or lung cells of patients should contribute to a significant amelioration of the symptoms by expression of the functional polypeptide in targeted tissues. Moreover, in cystic fibrosis studies have suggested that one would need to achieve expression of the CFTR gene product in only about 5% of lung epithelial cells in order to significantly improve the pulmonary symptoms.
Another application of gene therapy is vaccination. In this regard, the immunogenic product encoded by the polynucleotide introduced in cells of a vertebrate may be expressed and secreted or be presented by said cells in the context of the major histocompatibility antigens, thereby eliciting an immune response against the expressed immunogen. Functional polynucleotides can be introduced into cells by a variety of techniques resulting in either transient expression of the gene of interest, referred to as transient transfection, or permanent transformation of the host cells resulting from incorporation of the polynucleotide into the host genome.
Successful gene therapy depends on the efficient delivery to and expression of genetic information within the cells of a living organism. Most delivery mechanisms used to date involve viral vectors, especially adeno- and retroviral vectors. Viruses have developed diverse and highly sophisticated mechanisms to achieve this goal including crossing of the cellular membrane, escape from lysosomal degradation, delivery of their genome to the nucleus and, consequently, have been used in many gene delivery applications in vaccination or gene therapy applied to humans. The use of viruses suffers from a number of disadvantages: retroviral vectors cannot accommodate large-sized DNA (for example, the dystrophin gene which is around 13 Kb), the retroviral genome is integrated into host cell DNA and may thus cause genetic changes in the recipient cell and infectious viral particles could disseminate in the organism or in the environment and adenoviral vectors can induce a strong immune response in treated patients (Mc Coy et al., Human Gene Therapy 6 (1995), 1553-1560; Yang et al., Immunity 1 (1996), 433-442). Nevertheless, despite these drawbacks, viral vectors are currently the most useful delivery systems because of their efficiency.
Non-viral delivery systems have been developed which are based on receptor-mediated mechanisms (Perales et al., Eur. J. Biochem. 226 (1994), 255-266; Wagner et al., Advanced Drug Delivery Reviews 14 (1994), 113-135), on polymer-mediated transfection such as polyamidoamine (Haensler and Szoka, Bioconjugate Chem. 4 (1993), 372-379), dendritic polymer (WO 95/24221), polyethylene imine or polypropylene imine (WO 96/02655), polylysine (US-A-5 595 897 or FR 2 719 316) or on lipid-mediated transfection (Feigner et al., Nature 337 (1989), 387-388) such as DOTMA (Feigner et al., Proc. Natl. Acad. Sci. USA 84 (1987), 7413-7417), DOGS or Transfectam^{TM} (Behr et al., Proc. Natl. Acad. Sci. USA 86 (1989), 6982-6986), DMRIE or DORIE (Feigner et al., Methods 5 (1993), 67-75), DC-CHOL (Gao and Huang, BBRC 179 (1991), 280-285), DOTAP™ (McLachlan et al., Gene Therapy 2 (1995), 674-622) or Lipofectamine^{TM}. These systems present potential advantages with respect to large-scale production, safety, targeting of transfectable cells, low immunogenicity and the capacity to deliver large fragments of DNA. Nevertheless their efficienc*y in vivo* is still limited.

Finally, in 1990, Wolff et al. (Science 247 (1990), 1465-1468) have shown that injection of naked RNA or DNA, without a special delivery system, directly into mouse skeletal muscle results in expression of reporter genes within the muscle cells. This technique for transfecting cells offers the advantage of simplicity and experiments have been conducted that support the usefulness of this system for the delivery to the lung (Tsan et al., Am. J. Physiol. 268 (1995), L1052-L1056; Meyer et al., Gene Therapy 2 (1995), 450-460), brain (Schwartz et al., Gene Therapy 3 (1996), 405-411), joints (Evans and Roddins, Gene therapy for arthritis; In Wolff (ed) Gene therapeutics: Methods and Applications of direct Gene Transfer. Birkhaiser. Boston (1990), 320-343), thyroid (Sikes et al., Human Gen. Ther. 5 (1994), 837-844), skin (Raz et al., Proc. Natl. Acad. Sci. USA 91 (1994), 9519-9523) and liver (Hickman et al., Hum. Gene Ther. 5 (1994), 1477-1483). Nevertheless, Davis et al. (Human Gene Therapy 4 (1993), 151-159 and Human Mol. Genet. 4 (1993), 733-740) observed a large variability of expression due to nonuniform distribution of naked DNA injected into skeletal muscle *in vivo*. Only a small proportion of the muscle fibers (about 1-2%) are transfected and this level of gene transfer would be insufficient for the treatment of primary myopathies. The authors propose solutions in order to obtain an improvement of the efficiency of gene transfer (resulting in about 10% of transfected muscle fibers) by preinjecting muscles with a relatively large volume of hypertonic sucrose or with toxins, for example cardiotoxin isolated from snake, in order to stimulate regeneration of muscles. These methods, although promising, would not be applicable for human treatment.
Thus, the available delivery methods are not satisfactory in terms of safety or efficiency for their implementation in *in vivo* gene therapy.

Therefore, the technical problem underlying the present invention is the provision of improved methods and means for the delivery of nucleic acid molecules in gene therapy.

This technical problem is solved by the provision of the embodiments as defined in the claims.

Thus, the present invention relates to the use of a nuclease inhibitor for the preparation of a therapeutic composition for introducing a polynucleotide into a cell. It was surprisingly found that the addition of a nuclease inhibitor when transfecting a polynucleotide into vertebrate tissue leads to a dramatic improvement of the transfection efficiency. In particular, it was surprisingly found that if the polynucleotide is injected together with a nuclease inhibitor, e.g., into muscular tissue, the transfection is not only improved in the surrounding of the injection site but also in other areas of the muscle. Thus, the present invention preferably relates to the use of a nuclease inhibitor for the preparation of a pharmaceutical composition for an improved introduction of a polynucleotide into a cell. The term "improved introduction" in the scope of the present invention means, in this regard, a more efficient uptake of a polynucleotide by cells when a nuclease inhibitor is present compared to an introduction performed without a nuclease inhibitor. This can be determined by comparing the amount of the polynucleotide taken up without the use of a nuclease inhibitor and comparing this amount with the amount taken up by the cells when using a nuclease inhibitor under the same experimental conditions. Preferably, the improved introduction can be determined by a higher amount of expression of the polynucleotide transferred into the cells when using a nuclease inhibitor in comparison to a situation where no nuclease inhibitor is used.
Preferably, an improved introduction of the polynucleotide into the cell means that the uptake of the polynucleotide by cells is not only improved at the site of administration of the polynucleotide and nuclease inhibitor but is also improved in neighboring cells. Particularly preferred, an improved introduction means that the used nuclease inhibitor shows the same improving effect on the uptake of a polynucleotide by cells as does G-actin when compared to the administration of the polynucleotide without any nuclease inhibitor. The therapeutic compositions according to the present invention are particularly useful for the delivery of polynucleotides to cells or tissues of a subject in the scope of a genetherapeutic method but are not limited to such use. The term "gene therapy method" is preferably understood as a method for the introduction of a polynucleotide into cells either in vivo or by introduction into cells in vitro followed by re-implantation into a subject. "Gene therapy" in particular concerns the case where the gene product is expressed in a target tissue as well as the case where the gene product is excreted, especially into the blood stream.
In the scope of the present invention the term "introduction" means the transfer of the polynucleotide into a cell (transfection).

In the scope of the present invention the term "nuclease" means an enzyme with the capability to degrade nucleic acid molecules. Such nucleases encompass nucleases which can degrade single stranded nucleic acid molecules as well as nucleases which can degrade double stranded nucleic acid molecules. Furthermore, the nuclease can have the capability to degrade RNA or DNA. Preferably, it is a nuclease which degrades DNA. More preferably, the nuclease is a DNAse I, and particularly preferred a human nuclease.
A DNAse I in the scope of the present invention is to be understood as an endonuclease that hydrolyzes double-stranded or single-stranded DNA preferentially at sites adjacent to pyrimidine nucleotides. The product of this hydrolysis is a complex mixture of 5'-phosphate mono- and oligonucleotides. In the presence of Mg²⁺, a DNAse I attacks each strand of DNA independently and the sites of cleavage are distributed in a statistically random fashion. Furthermore, in the presence of Mn²⁺, DNAse I cleaves both strands of DNA at approximately the same site to yield fragments of DNA that are blunt-ended or that have protruding termini only one or two nucleotides in length.

In the scope of the present invention a nuclease inhibitor is defined by its capacity to act on a nuclease activity in a way that leads to a total or partial loss of the property of the nuclease to degrade a nucleic acid molecule. This capacity can be determined by incubating the potential inhibitor with the nuclease and with a nucleic acid molecule, which is normally degraded by the nuclease, under conditions which normally allow the nuclease to degrade the nucleic acid molecule and by determining whether the inhibitor represses or decreases the degradation of the nucleic acid molecule. The inhibitor can bind to the nuclease or can react with it. The inhibitor can be, for example, a chemical compound or a protein or fragment of a protein having nuclease inhibitor activity. Examples are antibodies or parts of antibodies which react specifically with a nuclease. Preferably, such an antibody is a monoclonal antibody.
It is possible for the person skilled in the art to find in the literature molecules described as nuclease inhibitors. Described are, for example, antibiotic compounds such as coumermycin or novobiocin, nalidixic or oxolinic acids (Fox and Studzinski, J. Histochem Cytochem. 30 (1982), 364-370), ciprofloxacin (CFL) or norfloxacin (Tempel and Ignatius, Arzneimittelforschung 42 (1992), 1031-1036) and aurintricarboxylic acid (ATA) (Benchokroum et al., Biochem. Pharmacol. 49 (1995), 305-313). More preferably, the nuclease inhibitor is not an acidic molecule and, if it is an acidic molecule, it is administered in a buffered neutral solution.
In a preferred embodiment the nuclease inhibitor is an inhibitor of a DNAse I. More preferably, it is a polypeptide or a fragment of a polypeptide which inhibits a DNAse I. In a particularly preferred embodiment the nuclease inhibitor is the globular form of actin (G-actin) (Harwell et al., J. Biol. Chem. 255 (1980), 1210-1220). Several publications have described the ability of G-actin to interact with a large number of actin binding proteins including DNAse I (Sheterline and Sparrow, Protein Profile 1 (1994), 1-121). G-actin binds DNAse I with high affinity and is a potent inhibitor (Kᵢ 1nM) of DNA hydrolytic activity (Lacks, J. Biol. Chem. 256 (1981), 2644-2648; Pinder and Gratzer, Biochemistry 21 (1982), 4886-4890). Based on these observations, Snabes et at. (J. Biol. Chem. 256 (1981), 6291-6295) have developed an immunoprecipitation assay based on DNAse I/actin binding using rabbit skeletal muscle actin or actin present in tissue and cell extracts. It is known that the interface between DNAse I and G-actin involves two exposed loops in subdomain II (residues P38 to S52) and IV (residues T194 to T203). Thus, a fragment of G-actin used in the scope of the present invention preferably comprises the residues forming these loops. The G-actin may be a naturally occurring form of G-actin, a modified G-actin (Carlier, Biochemistry 31 (1992), 300-309), a polypeptide complexed form (Peitsch et al., EMBO J. 12 (1993), 371-377) or a truncated form as long as the resulting polypeptide retains its ability to inhibit DNAse I activity. The G-actin may be, in principle, of any origin, preferably from vertebrates, more preferably from mammals, e.g. porcine, rabbit, bovine or human origin. G-actin is an ubiquitously expressed polypeptide and can be purified from mainly skeletal muscle and heart or produced by recombinant technology. G-actin is supplied for example by Sigma.

G-actin is known to have further activities, e.g. the capacity to bind divalent metal ions, such as calcium and magnesium ions, and the capacity to bind and hydrolyze ATP, which may cause or contribute to the observed effect of improved introduction of a polynucleotide into cells. Thus, also other proteins having these properties might be useful in the scope of the present invention.
In a preferred embodiment the prepared therapeutic composition in accordance with the present invention is in a form for administration into a vertebrate tissue. These tissues include those of muscle, skin, brain, lung, liver, spleen, bone marrow, thymus, heart, lymph, bone, cartilage, pancreas, kidney, gall bladder, stomach, intestine, testis, ovary, uterus, rectum, nervous system, eye, gland, connective tissue, blood, tumor etc. Cells where the improved transfection of a foreign polynucleotide would be obtained are those found in each of the listed target tissues (muscular cells, airway cells, hematopoïetic cells, etc.). The administration may be made by intradermal, subdermal, intravenous, intramuscular, intranasal, intracerebral, intratracheal, intraarterial, intraperitoneal, intravesical, intrapleural, intracoronary or intratumoral injection, with a syringe or other devices.
Transdermal administration is also contemplated, as are inhalation or aerosol administration.

In a preferred embodiment, the therapeutic composition is for the introduction into muscle tissue, more preferably, by intramuscular injection routes.

In a preferred embodiment, the invention provides the use of a nuclease inhibitor for the preparation of a therapeutic composition for improving transfection of a polynucleotide into a cell wherein said therapeutic composition is administered independently from a second administration consisting in administration of a composition containing at least one polynucleotide. According to a particularly preferred embodiment, the administration of said therapeutic composition is performed prior to said second administration. The therapeutic composition administration and second administration can be performed by different delivery routes (systemic delivery and targeted delivery, or targeted deliveries for example).

In a preferred embodiment, each should be done into the same target tissue and most preferably by injection.

In another preferred embodiment of the use according to the present invention, the therapeutic composition further comprises at least one polynucleotide. In a particularly preferred embodiment, the polynucleotide which is contained in the composition, contains and is capable of functionally expressing a gene in said cell.
The polynucleotide may be a DNA or RNA, single or double stranded, linear or circular, natural or synthetic, modified or not (see US 5525711, US 4711955 or EP-A 302 175 for modification examples). It may be, *inter alia*, a genomic DNA, a cDNA, an mRNA, an antisense RNA, a ribosomal RNA, a ribozyme, a transfer RNA or DNA encoding such RNAs. "Polynucleotides" and "nucleic acids" are synonyms with regard to the present invention. The polynucleotide may also be in the form of a plasmid or linear polynucleotide which contains at least one expressible sequence of nucleic acid that can generate a polypeptide, a ribozyme, an antisense RNA or another molecule of interest upon delivery to a cell. The polynucleotide can also be an oligonucleotide which is to be delivered to the cell, e.g., for antisense or ribozyme functions. The polynucleotide according to the present invention should preferably be understood as a naked polynucleotide (Wolff et al., Science 247 (1990), 1465-1468) or as a polynucleotide associated or complexed with a viral polypeptide or a cationic compound or with any component which can participate in the uptake of the polynucleotide into the cells (see Ledley, Human Gene Therapy 6 (1995), 1129-1144 for a review). Both DNA or RNA can be delivered to cells to form therein a polypeptide of interest. Preferably, the polynucleotide present in the therapeutic composition is in the form of plasmid DNA. If the polynucleotide contains the proper genetic information, it will direct the synthesis of relatively large amounts of the encoded polypeptide. When the polynucleotide delivered to the cells encodes an immunizing polypeptide, the use according to the invention can be applied to achieve improved and effective immunity against infectious agents, including intracellular viruses, and also against tumor cells. The genetic information necessary for expression by a target cell comprise all the elements required for transcription of said DNA into mRNA and for translation of mRNA into polypeptide. Transcriptional promoters suitable for use in various vertebrate systems are well known. For example, suitable promoters include viral promoters like RSV, MPSV, SV40, CMV or 7.5k, vaccinia promoter, inducible promoters, etc. The polynucleotide can also include intron sequences, targeting sequences, transport sequences, sequences involved in replication or integration. Said sequences have been reported in the literature and can be readily obtained by those skilled in the art. The polynucleotide can also be modified in order to be stabilized with specific components as spermine. According to the invention, the polynucleotide can be homologous or heterologous to the target cells into which it is introduced. Advantageously said polynucleotide encodes all or part of a polypeptide, especially a therapeutic or prophylactic polypeptide. A polypeptide is understood to be any translational product of a polynucleotide regardless of size, and whether glycosylated or not, and includes peptides and proteins. Therapeutic polypeptides include as a primary example those polypeptides that can compensate for defective or deficient proteins in an animal or human organism, or those that act through toxic effects to limit or remove harmful cells from the body. They can also be immunity conferring polypeptides which act as endogenous immunogens to provoke a humoral or cellular response, or both. Examples of polypeptides encoded by the polynucleotide are enzymes, hormones, cytokines, membrane receptors, structural polypeptides, transport polypeptides, adhesines, ligands, transcription factors, traduction factors, replication factors, stabilization factors, antibodies, more especially CFTR, dystrophin, factors VIII or IX, E6 or E7 from HPV, MUC1, BRCA1, interferons, interleukin (IL)2, IL-4, IL-6, IL-7, IL-12, GM-CSF (Granulocyte Macrophage Colony Stimulating Factor), the tk gene from Herpes Simplex type 1 virus (HSV-1), p53 or VEGF. The polynucleotide can also code for an antibody. In this regard, antibody encompasses whole immunoglobulins of any class, chimeric antibodies and hybrid antibodies with dual or multiple antigen or epitope specificities, and fragments, such as F(ab)₂, Fab', Fab including hybrid fragments and anti-idiotypes (US 4,699,880).

In another aspect the invention relates to a composition for the introduction of a polynucleotide into a cell, said composition comprising at least one polynucleotide and at least one nuclease inhibitor. Polynucleotide and nuclease inhibitor components are defined as above. Preferably, the composition furthermore comprises a pharmaceutically acceptable carrier.

In a preferred embodiment, the nuclease inhibitor contained in said composition is a DNAse inhibitor and even more preferred, a DNAse I inhibitor. In a particularly preferred embodiment, the nuclease inhibitor is G-actin or a fragment thereof, having the capability to inhibit a DNAse I.
According to the present invention, the amount of nuclease inhibitor in the compositions ranges preferably between 4x10⁻⁵ and 4 µg per µg of DNA, preferably between 4x10⁻⁴ and 2µg per µg of DNA. In a preferred embodiment, said composition comprises between 4x10⁻³ and 4x10⁻¹ µg of nuclease inhibitor per µg of DNA.

In another preferred embodiment, the polynucleotide which is contained in the composition, contains and is capable of functionally expressing, a gene in a cell, preferably in a vertebrate cell. One particularly preferred embodiment of the invention is a composition wherein said polynucleotide is naked. Nevertheless, the polynucleotide comprised in said composition can also be associated with viral polypeptides, or complexed with cationic components, more particularly with cationic lipids. In general, the concentration of polynucleotide in the composition is from about 0.1 µg/ml to about 20 mg/ml.

In a further preferred embodiment the composition further comprises at least one component selected from the group consisting of chloroquine, protic compounds such as propylene glycol, polyethylene glycol, glycerol, ethanol, 1-methyl L-2-pyrrolidone or derivatives thereof, aprotic compounds such as dimethylsulfoxide (DMSO), diethylsulfoxide, di-n-propylsulfoxide, dimethylsulfone, sulfolane, dimethylformamide, dimethylacetamide, tetramethylurea, acetonitrile or derivatives. The composition may also advantageously comprise a source of a cytokine which is incorporated in the form of a polypeptide or as a polynucleotide encoding the cytokine. Preferably, said cytokine is interleukin 10 (IL-10). According to a preferred embodiment, the composition comprises 5-15 % of DMSO and/or 0.001 to 1µg preferably 0.01 to 0.1 µg of IL-10.

In a further preferred embodiment the composition according to the invention can be used in a method for the therapeutic treatment of humans or animals. In this particular case, the composition according to the invention may also comprise a pharmaceutically acceptable injectable carrier. The carrier is preferably isotonic, hypotonic or weakly hypertonic and has a relatively low ionic strength, such as provided by a sucrose solution. Furthermore, it may contain any relevant solvents, aqueous or partly aqueous liquid carriers comprising sterile, pyrogen-free water, dispersion media, coatings, and equivalents. The pH of the pharmaceutical preparation is suitably adjusted and buffered.

In another aspect the present invention also relates to a process for introducing a polynucleotide into cells wherein said process comprises contacting said cells with at least one composition according to the invention. This process may be applied by direct administration of said composition to cells of the animal *in vivo*, or by *in vitro* treatment of cells which can be extracted from the animal and then re-introduced into the animal body (*ex vivo* process). According to the practice of the invention, targeted "cells" and "*in vivo* administration route" are defined as above described.

The present invention also relates to a process for introducing a polynucleotide into cells wherein said process comprises contacting the cells simultaneously or subsequently with a nuclease inhibitor and the polynucleotide. Preferably, the cells are first contacted with the nuclease inhibitor and afterwards with the polynucleotide. "Nuclease inhibitor", "polynucleotide" and the target cells are defined as above.
Preferably, muscle is used as a site for the delivery and expression of a polynucleotide in a number of therapeutic applications because animals have a proportionately large muscle mass which is conveniently accessed by direct injection through the skin. Accordingly, in a preferred case, the invention concerns a process for introducing a polynucleotide, preferably in naked form, into muscle cells *in vivo*, comprising the steps of administering *in vivo* at least a polynucleotide and at least a nuclease inhibitor, preferably G-actin, preferably intramuscularly, whereby the polynucleotide is introduced into muscle cells of the tissue. The polynucleotide may encode a therapeutic polypeptide that is expressed by the muscle cells and eventually secreted into the blood stream after the contacting step to provide therapy to the vertebrate. Similarly, it may encode an immunogenic polypeptide that is expressed by the muscle cells after the contacting step and which generates an immune response, thereby immunizing the vertebrate. One important aspect of the invention is a process for the treatment of muscular dystrophy wherein said polynucleotide operatively codes for dystrophin. Preferably, the composition is introduced into the muscle tissue.

The invention has been described in an illustrative manner, and it is to be understood that the terminology which has been used is intended to be in the nature of words of description rather than of limitation. Obviously, many modifications and variations of the present invention are possible in light of the above teachings. It is therefore to be understood that within the scope of the claims, the invention may be practiced otherwise than as specifically described.

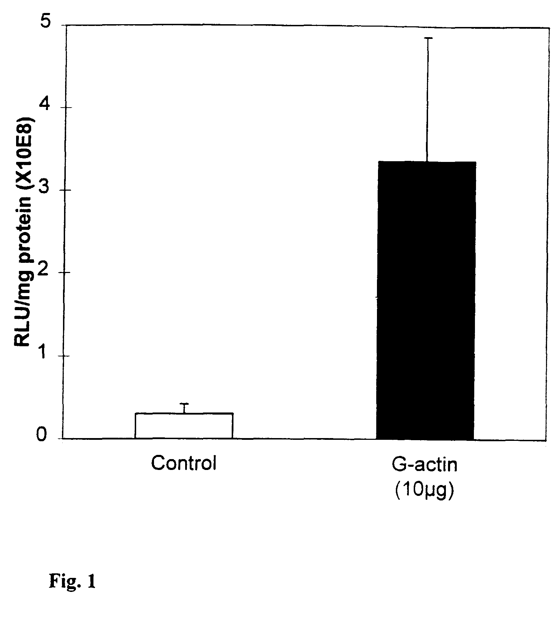

- **Figure 1**:: Effect of DNAse inhibitors on pTG11033 intramuscular transfection. Luciferase activity of mouse right and left tibialis anterior muscles measured 7 days after injection with 25µg plasmid added with NaCl 0.9% buffer (Control, empty bar) or with 10µg of G-actin (G-actin, full black bar). Bars are means of RLU (Relative Light Unit) per minute per mg proteins +/- s.e.m. of 8 determinations.

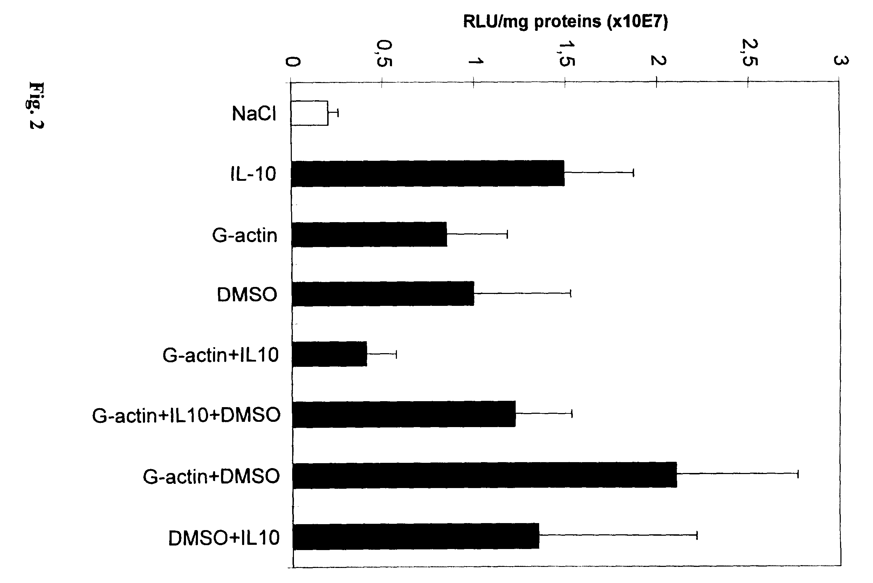

- **Figure 2**:: Combinations of adjuvants including G-actin to improve intramuscular transfer of the luciferase-plasmid (pTG11033). Bars are means of RLU per minute per mg proteins +/- s.e.m of 8 determinations. Luciferase activity was measured 7 days after plasmid injection into C57BL/10 mice (4 mice per group) added with either NaCl 0.9% (empty bars) or different combinations of adjuvants (black bars), NaCl 0.9%, IL-10 0.1µg, DMSO 10% final, G-actin 10 µg, G-actin + IL-10, or DMSO+G-actin+IL-10.

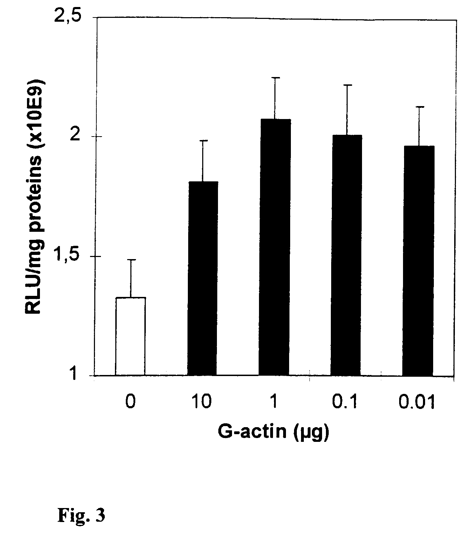

- **Figure 3**:: Dose/Response effect of G-Actin. Luciferase activity of right and left tibialis anterior muscles of 4 mice per group, 7 days after injection with plasmid added with NaCl 0.9% (empty bar) or different doses of G-actin (full black bars). Bars are means of RLU per minute per mg proteins +/- s.e.m. of 8 determinations.

The following examples illustrate the invention.

### MATERIAL AND METHODS

The following materials and methods are used in the examples.
***1. Plasmid/nuclease inhibitor composition intramuscular administration***
   Plasmids are prepared according to Bischoff et al., Analytical Biochemistry 254 (1997), 69-81. The tested nuclease inhibitors were mixed with the plasmid preparation (pTG11033: CMV promoter, β-globin intron, luciferase cassette; pTG11025: CMV promoter, β-globin intron, dystrophin cassette; both diluted in 0.9 % NaCl), prior to intramuscular injection. 25µg of plasmid are injected per muscle in 5 to 10 week-old C57BI/10 or *mdx* mice. The 2 *tibialis anterior* (right and left) muscles were injected (each muscle was considered as a sample, which means number of samples per condition = 2 x number of mice per condition).
***2. Muscle biopsies***
   One week after injection of the composition, mice were killed and the tibialis anterior muscles were retrieved and frozen. Based on the injected vector, either luciferase activity was determined in muscle extracts, or dystrophin expression was evaluated by immunohistochemistry.
***3. Luciferase measurement***
   Luciferase activity was quantified using a conventional measurement kit (Luciferase Assay System, Promega). Briefly, muscles were ground separately and diluted in 200 µl of reporter lysis buffer (Promega). 10 µl-samples were placed in the 96 well-plates and mixed with 100µl of substrate. Luciferase activity was expressed as number of RLU emitted per minute.
***4. Protein determination***
   Proteins were measured on 10µl samples using a VCA Protein Assay Kit (Pierce).
***5. Dystrophin immunohistochemistry***
   Muscle samples were frozen in liquid nitrogen-cooled isopentane and stored at -80°C. Immunofluorescence microscopy using anti-dystrophin antibody was performed as follows: serial cryostat transverse sections (5-8 µm) of unfixed muscles were prepared on glass slices, dipped in PBS buffer with 1% mouse serum and incubated for 30 min at room temperature, for saturation of the non specific binding sites. After rinsing (3 times, 5 mm) in PBS buffer, slices were dipped in PBS buffer containing 1/500 dilution of anti-dystrophin monoclonal antibody (MANDRA-1, Sigma) and incubated for 90 min at room temperature. Slices were rinsed 3 times for 5 mm in PBS, then incubated for 30 mm at room temperature with biotin-F(ab')₂ goat anti-mouse IgG (H+L) diluted 1/500 in PBS. After rinsing (3 times, 5 mm in PBS), preparations were incubated 30 mm at room temperature with 1/2000 Streptavidin-FITC (1 mol Streptavidin/4 mol Biotin). Slices were then rinsed and mounted with Mowiol for microscopic evaluation.

### Example 1

### Effect of DNAse inhibitor on pTG11033 intramuscular transfection

In this example, 10µg of G-actin were added to pTG11033 (25µg/muscle) in a total volume of 30 µl to be injected. 4 C57BL/10 adult mice (male and female) per group have been injected in tibialis anterior muscles. The control experiment is performed according to the same condition except that no G-actin is added. The results are presented in Figure 1. They show that the addition of G-actin leads to a significant increase of intramuscular transfection of the plasmid in muscular cells. This example shows that G-actin increases significantly (about 12 times in the present example) gene transfer into skeletal muscle as evidenced by luciferase activity measurement of the injected muscles 7 days after plasmid administration.

### Example 2

### Combinations of adjuvants including G-actin to improve ( i.m.) transfer of dystrophin-plasmid (pTG11025)

It was tried to enhance the observed improvement by adding other components to the composition of the invention. In this example, 6 *mdx* mice were injected (final volume injected: 35 µl) with pTG11025 plasmid preparation added with:
(1) NaCl 0.9%;
(2) IL-10 0.1µg;
(3) DMSO 10% final;
(4) G-actin 10 µg;
(5) G-actin 10 µg + IL-10 0.1µg; or
(6) DMSO 10% final + G-actin 10 µg + IL-10 0.1µg

In these experiments, notexin-induced necrosis-regeneration was carried out 3 days prior to plasmid injection (Lefaucheur et Sebille, Neuromuscul. Disord. 5 (1995), 501-509).

Tibialis anterior muscles were collected 7 days after injection, and histological analysis of the transfected tibialis anterior muscle have been conducted. The results (Table I) show the following order of efficiency: 6>4>5>3>2>1

**Table I**

| **Tested molecules** | **Number of dystrophin-positive fibers per cryosection** |
|---|---|
| DMSO+G-actin+IL-10 | 89.3 +/- 19.7 |
| G-actin | 71.0 +/- 6.0 |
| G-actin + IL-10 | 44.5 +/- 6.9 |
| DMSO | 37.8 +/- 5.5 |
| IL-10 | 21.7 +/- 1.9 |
| NaCl | 3.3 +/- 0.7 |

Values are mean +/- sem of up to 4 determinations par muscle (obtained from the serial sections of the whole muscles).
In this experiment the best condition (DMSO+G-actin+IL-10) led to around 15% of dystrophin-positive fibers in the mdx tibialis anterior muscles. Interestingly, histological analysis shows that those positive fibers were not localized to a fraction of the muscle (i.e. along the needle track), but homogeneously in the transversal sections. Example 2 (as well as example 3) show that combinations of G-actin and other compounds may act synergistically on gene transfer.
In a second experiment *mdx* mice were 3 times injected intramuscularly with pTG11025 (dystrophin) plus DMSO, G-actin and IL-10 after notexin-induced muscle regeneration. Plasmid-adjuvant injections were repeated 3 times in raw on a daily basis and dystrophin expression (immunohistochemistry) was evaluated 7 days after the last injection. In this case up to 20% of dystrophin-positive fibers were found in the injected muscles.

### Example 3

### Combinations of adjuvants including G-actin to improve intracellular transfer of luciferase- plasmid (pTG11033)

The above described experiments have been reproduced using the pTG11033 (luciferase) plasmid. Luciferase activity was measured 7 days after injection of the composition in C57BL/10 mice (4 mice per group).
The data show (Figure 2) that injection of naked DNA in accordance with the present invention (intramuscularly) produced improved expression in muscle which is not limited to the injection site. Thus, one of the fundamental differences between the present invention and the prior art methods is that the present invention results in an increased non localized gene expression in muscle cells and thus provides the possibility for improving gene expression which is not possible with prior art methods. Furthermore, because of the need for fewer injections for equivalent efficiency, application of the present invention is likely to be better tolerated by patients.

### Example 4

### Dose/Response effect of G-Actin

25 µg of plasmid DNA (pTG11033 preparation at 2 mg/ml in 0.9% NaCl) was added with various dilutions (in 0.9% NaCl) of G-actin at final concentrations ranging from 0.01 to 10 µg per 30 µl (final volume), 4 mice per condition. Injections were performed in both right and left *tibialis anterior.* Luciferase activity was measured in muscles that were collected 7 days after plasmid injection.
As shown by Figure 3, luciferase activity is increased in the muscle that has been injected with plasmid added with G-actin even at low concentrations. Maximal effect seems to be obtained at G-actin concentrations of 0.1 to 1 µg/25 µg plasmid DNA. Bars are mean +/- sem of 8 values per condition.

## Claims

1. Use of a nuclease inhibitor for the preparation of a therapeutic composition for the introduction of a polynucleotide into a cell.

2. The use of claim 1, wherein said nuclease inhibitor is a deoxyribonuclease (DNAse) inhibitor, preferably a DNAse I inhibitor.

3. The use of claim 2, wherein said nuclease inhibitor is G-actin or a fragment thereof capable of inhibiting DNAse I activity.

4. The use of claim 3, wherein said G-actin is of porcine, rabbit, bovine or human origin.

5. The use of any one of claims 1 to 4, wherein said therapeutic composition is for administration into a vertebrate target tissue.

6. The use of claim 5, wherein said administration is made by intradermal, subdermal, intravenous, intramuscular, intranasal, intracerebral, intratracheal, intraarterial, intraperitoneal, intravesical, intrapleural, intracoronary or intratumoral injection.

7. The use of claim 5, wherein said administration is made into the lung by inhalation or aerosol administration.

8. The use of claim 5, wherein said target tissue is muscle.

9. The use of any one of claims 5 to 8, wherein the administration of the nuclease inhibitor is performed independently from a second administration consisting in administration of a composition containing at least one polynucleotide into the same target tissue.

10. The use of claim 9, wherein the administration of the nuclease inhibitor is performed prior to said second administration.

11. The use of any one of claims 1 to 8, wherein said therapeutic composition further comprises at least one polynucleotide.

12. The use of any one of claims 1 to 11, wherein said polynucleotide contains a gene and is capable of functionally expressing said gene in said cell.

13. A composition for introducing a polynucleotide into a cell, said composition comprising at least one polynucleotide and at least one nuclease inhibitor.

14. The composition of claim 13, wherein said nuclease inhibitor is a DNAse inhibitor, preferably a DNAse I inhibitor.

15. The composition of claim 14, wherein said nuclease inhibitor is G-actin or a fragment thereof with the capability to inhibit DNAse I activity.

16. The composition of claim 15, wherein said G-actin or fragment thereof is porcine, rabbit, bovine or human origin.

17. The composition of claim 15 or 16, wherein said composition contains between 4x10⁻⁵ and 4 µg, preferably between 4x10⁻⁴ and 2µg, and more preferably comprises between 4x10⁻³ and 4x10⁻¹ µg of nuclease inhibitor per µg of DNA.

18. The composition of any one of claims 13 to 17, wherein the polynucleotide concentration ranges from about 0.1 µg/ml to about 20 mg/ml.

19. The composition of any one of claims 13 to 18, wherein said polynucleotide contains a gene and is capable of functionally expressing said gene in said cell.

20. The composition of claim 18, wherein said gene encodes all or part of dystrophin or cystic fibrosis transmembrane conductance regulator (CFTR) polypeptides.

21. The composition of any one of claims 13 to 20, wherein said cell is a vertebrate cell.

22. The composition of any one of claims 13 to 21, wherein said polynucleotide is naked.

23. The composition of any one of claims 13 to 21, wherein said polynucleotide is associated with viral polypeptides.

24. The composition of any one of claims 13 to 21, wherein said polynucleotide is complexed with cationic components, more preferably with cationic lipids.

25. The composition of any of claims 13 to 24, wherein said composition further comprises at least one component selected from the group consisting of chloroquine, protic compounds such as propylene glycol, polyethylene glycol, glycerol, ethanol, 1-methyl L -2-pyrrolidone or derivatives, aprotic compounds such as dimethylsulfoxide (DMSO), diethylsulfoxide, di-n-propylsulfoxide, dimethylsulfone, sulfolane, dimethylformamide, dimethylacetamide, tetramethylurea, acetonitrile or derivatives, cytokines, preferably interleukin 10 (IL-10).

26. The composition of claim 25, wherein said composition comprises 5-15 % of DMSO and/or from about 0.001 to about 1µg preferably from about 0.01 to about 0.1 µg of IL-10.

27. The composition of any of claims 13 to 26 for use in a method for the therapeutic treatment of the human or animal body.

28. The composition of claim 27, wherein said composition further comprises a pharmaceutically acceptable injectable carrier.

29. A process for introducing a polynucleotide into cells wherein said process comprises contacting said cells with at least one composition of any one of claims 13 to 28.

30. A process for introducing a polynucleotide into cells wherein said process comprises contacting the cells simultaneously or subsequently with a nuclease inhibitor and the polynucleotide.

31. The process of claim 30, wherein the cells are first contacted with the nuclease inhibitor and subsequently with the polynucleotide.
